# FASCICULE DE BREVET EUROPEEN

(11) **EP 1 133 295 B1**
(45) Date de publication et mention de la délivrance du brevet: **03.09.2003**
(21) Numéro de dépôt: 99956108.7
(22) Date de dépôt: 22.11.1999
(51) Int. Cl.: A61K 31/48, A61P 21/00, A61P 25/00

(54) **UTILISATION DE LA NICERGOLINE DANS LE TRAITEMENT DE LA SPASTICITE**
VERWENDUNG VON NICERGOLINE ZUR BEHANDLUNG DER SPASTIZITÄT
USE OF NICERGOLINE FOR TREATING SPASMODISM

(30) Priorité: 24.11.1998 FR 9814794
(43) Date de publication de la demande: 19.09.2001
(73) Titulaire: Aventis Pharma S.A., 92160 Antony (FR)
(72) Inventeur: DIB, Michel, F-75013 Paris (FR); MEININGER, Vincent, F-75006 Paris (FR)
(74) Mandataire: Rousseau, Pierrick Edouard
(86) Numéro de dépôt international: FR9902866
(87) Numéro de publication internationale: WO00030642

(56) Documents cités:
- EP-A- 0 602 619
- DE-A- 4 240 798
- SANDERINK G J ET AL: "Involvement of human CYP1A isoenzymes in the metabolism and drug interactions of riluzole in vitro." JOURNAL OF PHARMACOLOGY AND EXPERIMENTAL THERAPEUTICS, (1997 SEP) 282 (3) 1465-72., XP002112642
- MARTINET M. ET AL: "Pharmacokinetics and metabolism of riluzole." DRUGS OF TODAY, (1997) 33/8 (587-594)., XP002112643
- O. ELWAN ET AL.: "Ergoloids and Ischaemic Strokes; Efficacy and Mechanism of Action" JOURNAL OF INTERNATIONAL MEDICAL RESEARCH, vol. 23, no. 3, 1995, pages 154-166, XP002112644
- K. TAKAHASHI ET AL.: "Nicergoline inhibits T-type ca2+ channels in rat isolated hippocampal CA1 pyramidal neurones" BRITISH JOURNAL OF PHARMACOLOGY, vol. 100, no. 4, 1990, pages 705-710, XP002112645
- DATABASE WPI Section Ch, Week 9408 Derwent Publications Ltd., London, GB; Class B02, AN 94-061991 XP002112646 & JP 06 016555 A (TANABE SEIYAKU CO), 25 janvier 1994 (1994-01-25)

## Description

La présente invention concerne l'utilisation de la nicergoline traitement de la spasticité pyramidale des maladies neurologiques comportant une atteinte de la voie pyramidale.

La spasticité fait partie des traits cliniques de maladies neurologiques ayant en commun une atteinte de la voie pyramidale comme par exemple la sclérose latérale amyotrophique, l'atrophie musculaire spinale progressive, l'atrophie musculaire infantile, la sclérose latérale primaire, les accidents vasculaires cérébraux.

La nicergoline ou (8β)-10-méthoxy-1,6-diméthylergoline-8-méthanol-5-bromonicotinate (Sermion®) présente notamment des propriétés α-bloquantes, α2-adrénolytiques (CARPENE C. et coll., J. Pharmacol, 14, 57-66 (1983)), antiischémiques (CAHN R. et coll., Chem. Abstracts, 107, 228784x (1987); UEDAT et coll., Chem. Abstracts, 118, 225224f (1993)), anticalciques (TAKAHASHI K. et coll., Br. J. Pharmacol., 100, 705-710 (1990)), antioxydantes (TANAKA M. et coll., Neurosci. Let., 248, 67-72 (1998)), antithrombotiques, (Chem. Abstracts 105, 54314k (1986)). Elle améliore la capacité d'apprentissage et la mémoire (Chem. Abstracts, 113, 52358u (1990); Chem. Abstracts, 111, 108396h, 1989; Chem. Abstracts, 109, 86208c, 1988; Chem. Abstracts, 106, 12788e, 1987; Chem. Abstracts, 115, 198237s, 1991).

Il a maintenant été trouvé que la nicergoline diminue la spasticité pyramidale des maladies neurologiques comportant une atteinte de la voie pyramidale et, notamment, de la spasticité lors de la sclérose latérale amyotrophique, de l'atrophie musculaire spinale progressive, de l'atrophie musculaire infantile, de la sclérose latérale primaire, des accidents vasculaires cérébraux.

La sclérose latérale amyotrophique (SLA) également connue sous le nom de maladie de CHARCOT et maladie de LOU GEHRIG a été décrite pour la première fois par CHARCOT en 1865. La SLA est une maladie mortelle résultant de la dégénérescence des motoneurones. La maladie s'accompagne d'une paralysie progressive, conduisant à la perte des fonctions motrices et respiratoires puis à la mort dans un délai de deux à huit ans après l'apparition des premiers symptômes.

La raideur fait partie des traits cliniques caractéristiques de cette affection. Son degré est variable et il est habituel de constater l'existence de patients très raides s'opposant à des patients peu ou pas raides.

Cette raideur est généralement assimilée à l'hypertonie (ou contracture ou spasticité) du syndrome pyramidal et il est habituel de la considérer comme due à l'atteinte exclusive de la voie pyramidale.

Cliniquement, cependant, il n'est pas toujours facile de rapporter à la seule atteinte pyramidale, la raideur observée.

En effet, il faut distinguer la raideur pyramidale (ou contracture ou spasticité) de la raideur extrapyramidale (ou rigidité).

La contracture pyramidale (augmentation du tonus moteur dit tendino-réflexes de Ch. Foix) est cliniquement élastique, elle se renforce à mesure que les points d'insertion musculaires se séparent, elle s'exagère avec la vitesse de déplacement du membre et elle restitue au membre mobilisé l'attitude primitive. Elle prédomine sur les fléchisseurs aux membres supérieurs et les extenseurs aux membres inférieurs, avec une prédominance sur les muscles distaux.

La rigidité extrapyramidale (exagération du tonus plastique dit posturo-réflexe de Ch. Foix), au contraire, est une sensation de résistance élastique cireuse, qui s'atténue par la mobilisation passive, douce, répétée. Elle touche tous les muscles, prédominant sur les muscles proximaux (rhizoméliques). Le tonus des membres s'exagère lors du raccourcissement passif du muscle définissant l'exaltation des réflexes de posture de Ch. Foix (rigidité de fixation de Strümpell). Elle tend à fixer le membre dans la position que l'observateur lui a imprimé. Elle se rapproche par là des phénomènes de catatonie et de catalepsie.

Enfin, il faut associer à ces modifications du tonus, la rigidité oppositionnelle ou de Gegenhalten traduisant plus une atteinte frontale. Elle est proche de la rigidité extrapyramidale mais s'en différencie par son caractère fluctuant et par son exagération lorsque le patient cherche à se relaxer.

Un certain nombre de données plaide en faveur de l'atteinte du système extrapyramidal chez les patients atteints de SLA.

Outre l'association connue de SLA et de maladie de Parkinson (notamment dans le complexe anatomo-clinique dit de Guam), différents travaux anatomo-cliniques ont objectivé une atteinte des noyaux gris centraux. Dès 1925, I. BERTRAND et L. Van BOGAERT (Rev. Neurol., 32, 779-806 (12925)) rapportent une atteinte diffuse du cortex cérébral et des noyaux gris centraux chez les patients atteints de SLA. En 1972, P. CASTAIGNE et coll. (Rev. Neurol., 127, 401-414 (1972)), notent une atteinte des noyaux gris centraux dans 16 cas sur 19 de SLA «atypiques», bien que chez ces patients, il ne semblait pas exister de signes extrapyramidaux cliniques. Des cas isolés de SLA avec atteinte de la substantia nigra ont également été rapportés (S.M. CHOU, Dekker pub. p133-181 (1992). Utilisant le PET scan avec la 6-fluorodopa, TAKAHASHI et coll. (J. Neural. Transm., 5, 17-26 (1993), ont montré une diminution de la capture de la fluorodopa, chez tous les patients en dépit, de l'absence de signe clinique net d'atteinte du système extrapyramidal. Cette diminution semble s'accentuer progressivement avec le temps au cours de la maladie.

L'expérience clinique montre que la plupart des patients raides ont une raideur mixte : pyramidale et extrapyramidale. Un argument important en faveur d'une origine probablement mixte de la raideur observée chez les patients atteints de SLA, est que ce symptôme est peu ou pas accessible aux traitements myorelaxants habituellement utilisés dans la spasticité pyramidale (baclofène, benzodiazépines, dantrolène).

La distinction entre rigidité et spasticité n'a pas seulement une importance descriptive, mais a aussi une importance dans la prise en charge de ces patients. En effet la raideur est un facteur probablement « favorable » dans le pronostic vital mais certainement « défavorable » dans le pronostic fonctionnel puisqu'elle est peu ou pas accessible aux myorelaxants habituels, même au prix d'une augmentation considérable de doses (parfois 180 mg/jour de baclofène).

Une étude descriptive portant sur 9 patients atteints d'une SLA a été réalisée. Le critère principal défini est la raideur, mesurée à l'aide d'une échelle analogique visuelle (EAV), de 100 mm, ou le sujet cote en autoévaluation l'importance de la raideur perçue.

A ce jour, seul le riluzole (2-amino-6-trifluorométhoxybenzothiazole) est commercialisé sous le nom de RILUTEK^{R} pour le traitement de la sclérose latérale amyotrophique. Le riluzole permet principalement de ralentir la progression de la maladie mais n'a aucun effet sur la spasticité.

Les patients reçoivent 100 mg/jour de riluzole et 15 mg IV de nicergoline le premier jour et de 30 mg IV les 4 jours suivants. La durée de perfusion était supérieure à 4 heures pour éviter tout risque d'hypotension.

La mesure a été effectuée avant et après administration de nicergoline.

Les résultats sont les suivants (Moyenne ± Déviation Standard, en mm) :
La raideur avant administration de nicergoline est de 67,33 ± 25
La raideur 4 jours après (de 4 à 8 j) est de 48,11 ± 19
La différence intra sujet est de 19,22 ± 20,5 (95 % I= 3,44+35; range = -8 à + 57)
La comparaison à une différence nulle est significative (t = 2,809 ; p (2 α) = 0,0229)

Le test d'écart à une distribution normale est non significatif.

La présente demande concerne également l'utilisation de l'association de riluzole ou un sel du riluzole pharmaceutiquement acceptable et de nicergoline dans le traitement de la spasticité pyramidale des maladies neurologiques comportant une atteinte de la voie pyramidale et, notamment, de la -spasticité lors de la sclérose latérale amyotrophique, de l'atrophie musculaire spinale progressive, de l'atrophie musculaire infantile, de la sclérose latérale primaire, des accidents vasculaires cérébraux.

La nicergoline peut être préparée selon le brevet US3228943.

Le riluzole peut être préparé selon le procédé décrit dans le brevet EP 50551.

Comme sels pharmaceutiquement acceptables du riluzole peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

La nicergoline peut être employée par voie orale, parentérale ou rectale.

L'association nicergoline et riluzole peut être employée par voie orale, parentérale ou rectale soit simultanément soit séparément soit de manière étalée dans le temps.

La présente invention concerne également les compositions pharmaceutiques comprenant la nicergoline pure ou sous forme d'une association avec un ou plusieurs diluants et/ou adjuvants compatibles et pharmaceutiquement acceptables et/ou éventuellement en association avec un autre produit pharmaceutiquement compatible et physiologiquement actif et éventuellement en association avec le riluzole ou un de ses sels pharmaceutiquement acceptables.

Comme compositions solides pour administration orale, peuvent être utilisés des comprimés, des pilules, des poudres (capsules de gélatine, cachets) ou des granulés.

Dans ces compositions, les principes actifs sont mélangés à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

La présente invention concerne également l'utilisation de la nicergoline soit seule soit associée au riluzole ou un de ses sels pharmaceutiquement acceptables pour la préparation de médicaments destinés au traitement de la spasticité pyrarnidale des maladies neurologiques comportant une atteinte de la voie pyramidale et notamment de la sclérose latérale amyotrophique, de l'atrophie musculaire spinale progressive, de l'atrophie musculaire infantile, de la sclérose latérale primaire, des accidents vasculaires cérébraux.

Les doses dépendent de l'effet recherché, de la durée du traitement et de la voie d'administration utilisée; elles sont généralement de 15 à 30 mg par jour par voie orale pour un adulte avec des doses unitaires de 5 à 10 mg de nicergoline.

Lorsque l'association nicergoline et riluzole est utilisée, les doses sont généralement de 10 à 400 mg par jour par voie orale pour un adulte avec des doses unitaires allant de 10 à 200 mg de riluzole et de 15 à 30 mg par jour par voie orale pour un adulte avec des doses unitaires de 5 à 10 mg denicergoline.

D'une façon générale, le médecin déterminera la posologie appropriée en fonction de l'âge, du poids et de tous les autres facteurs propres au sujet à traiter.

## Revendications

1. Utilisation de la nicergoline pour la préparation de médicaments destinés au traitement de la spasticité pyramidale des maladies neurologiques comportant une atteinte de la voie pyramidale.

2. Utilisation selon la revendication 1 pour laquelle la spasticité des maladies neurologiques comportant une atteinte de la voie pyramidale est la spasticité de la sclérose latérale amyotrophique, de l'atrophie musculaire spinale progressive, de l'atrophie musculaire infantile, de la sclérose latérale primaire ou des accidents vasculaires cérébraux.

3. Utilisation selon la revendication 1 ou 2 dans laquelle la nicergoline est en association avec le riluzole ou un de ses sels pharmaceutiquement acceptables.

4. Utilisation selon la revendication 1 ou 2 pour la préparation d'un médicament contenant 5 à 10 mg de nicergoline.

5. Utilisation selon la revendication 3 pour la préparation d'un médicament contenant 5 à 10 mg de nicergoline et 10 à 200 mg de riluzole.

## Patentansprüche

1. Verwendung von Nicergolin zur Herstellung von Arzneimitteln, die zur Behandlung der pyramidalen Spastik neurologischer Erkrankungen, die einen Befall der Pyramidenbahn umfassen, bestimmt sind.

2. Verwendung nach Anspruch 1, bei der die Spastik neurologischer Erkrankungen, die einen Befall der Pyramidenbahn umfassen, die Spastik der amyotrophen Lateralsklerose, der progressiven spinalen Muskelatrophie, der Kindermuskelatrophie, der primären Lateralsklerose oder bei Schlaganfällen ist.

3. Verwendung nach Anspruch 1 oder Anspruch 2, wobei Nicergolin in Kombination mit Riluzol oder einem seiner pharmazeutisch annehmbaren Salze vorliegt.

4. Verwendung nach Anspruch 1 oder Anspruch 2 zur Herstellung eines Arzneimittels, das 5 bis 10 mg Nicergolin enthält.

5. Verwendung nach Anspruch 3 zur Herstellung eines Arzneimittels, das 5 bis 10 mg Nicergolin und 10 bis 200 mg Riluzol enthält.

## Claims

1. Use of nicergoline for the preparation of medicinal products for the treatment of the pyramidal spasticity of neurological diseases involving an attack of the pyramidal pathway.

2. Use according to Claim 1, for which the spasticity of neurological diseases involving an attack of the pyramidal pathway is the spasticity of amyotrophic lateral sclerosis, progressive spinal muscular atrophy, infantile muscular atrophy, primary lateral sclerosis or cerabrovascular accidents.

3. Use according to Claim 1 or 2, in which nicergoline is in combination with riluzole or a pharmaceutically acceptable salt thereof.

4. Use according to Claim 1 or 2, for the preparation of a medicinal product containing 5 to 10 mg of nicergoline.

5. Use according to Claim 3, for the preparation of a medicinal product containing 5 to 10 mg of nicergoline and 10 to 200 mg of riluzole.
